# EUROPEAN PATENT APPLICATION

(11) **EP 3 641 062 A1**
(43) Date of publication of application: **22.04.2020**
(21) Application number: 18201007.4
(22) Date of filing: 17.10.2018
(51) Int. Cl.: H01R 4/2425, H05K 1/11, A61B 8/00, A61B 5/00, B06B 1/02

(54) **ELECTRIC COMPONENT OF AN INTERVENTIONAL MEDICAL DEVICE**

(71) Applicant: Koninklijke Philips N.V., 5656 AG Eindhoven (NL)
(72) Inventor: WEEKAMP, Johannes Wilhelmus, 5656 AE Eindhoven (NL); HENNEKEN, Vincent Adrianus, 5656 AE Eindhoven (NL); LOUWERSE, Marcus Cornelis, 5656 AE Eindhoven (NL); JACOBS, Egbertus Reinier, 5656 AE Eindhoven (NL)
(74) Representative: de Haan, Poul Erik

(57) **Abstract**

The present invention relates to electric components of interventional devices. In order to provide further miniaturization of medical interventional devices, an electric component (10) of an interventional device is provided. The component comprises a flat carrier base (12), at least one electric circuit (14) provided on a substrate (16) and at least one electric wire (18) connected to the electric circuit by a wire connection (20). The substrate is attached to the carrier base. Further, for the wire connection, the carrier base is provided with at least one opening (22) that is provided at least partly with a conductive edge portion (24), which edge portion is connected to the at least one electric circuit. Still further, the substrate is provided with at least one recess (26) aligned with the location of the at least one opening in the carrier base. Furthermore, an end portion of the wire is arranged in the at least one recess; and the end portion of the wire is conductively coupled to the conductive edge portion by electrically conductive material (30).

## Description

### FIELD OF THE INVENTION

The present invention relates to an electric component of an interventional device, to an interventional device for at least partly insertion into a subject and to a method for manufacturing an electric component of an interventional device.

### BACKGROUND OF THE INVENTION

In interventional medical devices, electric components are provided, e.g. integrated in the distal end of a catheter or guidewire. As an example, an electric circuit of a sensor in the end portion may be arranged. Because such interventional devices are provided for insertion into a vessel structure of a subject, the dimensions of the integrated electric components should be small. For example, WO 2018/095991 A1 relates to electrical connections to miniature sensors. As a further example, WO 2015/077449 A1 relates to semiconductor sensor chips in catheters. However, it has been shown that an increasing demand exists to further reduce the size of sensors e.g. for inserting a catheter distal end in smaller vessel structures.

### SUMMARY OF THE INVENTION

There may thus be a need to provide further miniaturization of medical interventional devices.

The object of the present invention is solved by the subject-matter of the independent claims; further embodiments are incorporated in the dependent claims. It should be noted that the following described aspects of the invention apply also for the electric component of an interventional device, for the interventional device for at least partly insertion into a subject and for the method for manufacturing an electric component of an interventional device.

According to the present invention, an electric component of an interventional device is provided. The component comprises a flat carrier base, at least one electric circuit provided on a substrate and at least one electric wire connected to the electric circuit by a wire connection. The substrate is attached to the carrier base. For the wire connection, the carrier base is provided with at least one slit opening that is provided at least partly with a conductive edge portion, which edge portion is connected to the at least one electric circuit. The substrate is provided with at least one recess aligned with the location of the at least one slit opening in the carrier base. Further, an end portion of the wire is arranged in the at least one recess. Furthermore, the end portion of the wire is conductively coupled to the conductive edge portion by soldering.

As a result, the size of the electric component is kept to a minimum and thus enables smaller sized distal ends of interventional devices.

According to an example, the substrate of the at least one electric circuit is attached to the carrier base on a first side and the at least one electric wire is connected to the carrier base also on the first side.

According to an example, the carrier base comprises a flexible layer and conductor paths attached to the flexible layer.

For example, a flexible carrier plate can be adjusted to different forms of an interventional device, e.g. due to guidewire bending.

According to an example, the substrate of the electric circuit is a rigid substrate made from silicon.

According to an example, the end portion of the wire is provided with a circumferentially enclosing electric insulation, which is removed in alignment with the respective slit opening on a side of the wire facing the slit opening.

According to an example, the at least one recess is arranged in line with, but displaced to the at least one slit opening. Further, the wire first extends through the recess and then reaches the slit opening. Still further, the wire is provided with a circumferential layer of electric insulation in a portion within the recess. Furthermore, the wire is mechanically coupled with an adhesive to edge portions of the substrate forming the recess.

According to an example, the at least one slit opening is arranged in line with the at least one recess, wherein the recess is having a longer extension than the slit opening such that a first part of the recess is provided with the slit opening in the carrier base and a second part is provided without a slit opening in the carrier base. Further, the wire is provided with an insulated section having a circumferential layer of electric insulation in a portion within the second part of the recess, in which insulated section the wire is mechanically coupled with an adhesive to edge portions of the substrate forming the recess. Still further, the wire is provided with a stripped end section without insulation in a portion within the first part of the recess, in which stripped end section the wire is connected by the soldering.

According to an example, the electric component relates to at least one of the group of a sensor, a measurement unit and an application unit for an interventional device.

According to the present invention, also an interventional device for at least partly insertion into a subject is provided. The device comprises an elongated main body structure and an electric component provided according to one of the preceding examples. The main body structure comprises a distal part, or distal end, for insertion into a subject, for example into vessels of a vascular structure. Further, the electric component is attached to the distal part of the main body structure.

According to the present invention, also a method for manufacturing an electric component of an interventional device is provided. The method comprises the following steps:
a) providing a flat carrier base with at least one electric circuit provided on a substrate, the substrate circuit being attached to the carrier base; wherein the carrier base is provided with at least one slit opening that is provided at least partly with a conductive edge portion, which edge portion is connected to the at least one electric circuit; and wherein the substrate is provided with at least one recess aligned with the location of the at least one slit opening in the carrier base;
b) providing at least one electric wire for connection with the electric circuit;
c) arranging an end portion of the at least one electric wire in the at least one recess; and
d) soldering the end portion of the wire to the conductive edge portion to conductively couple the end of the wire and the conductive edge portion.

According to an example, in step c), the end portion of the wire is provided with a circumferentially enclosing electric insulation. Further, before step d), the insulation is removed in alignment with the respective slit opening on a side of the wire facing the slit opening.

According to an aspect, an electric connection of an electrical circuit is provided with a cable connection that is connected by soldering via a slit opening in a carrier plate. The soldering is supplied so-to-speak from the other side. An end portion of the cable runs parallel to the substrate in a recess of the substrate provided for the electric circuit.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 shows an example of an electric component of an interventional device in a perspective view.
Fig. 2 shows a cross section of the electric component of Fig. 1.
Fig. 3 shows manufacturing stages of an example of an electric component.
Fig. 4 shows manufacturing stages of another example of an electric component.
Fig. 5a shows manufacturing stages of a further example of an electric component in a first viewing direction.
Fig. 5b shows the manufacturing stages of Fig. 5a in a second viewing direction.
Fig. 6 shows an example of an interventional device for at least partly insertion into a subject in the context of a medical examination setup.
Fig. 7 shows steps of an example of a method for manufacturing an electric component of an interventional device.

### DETAILED DESCRIPTION OF EMBODIMENTS

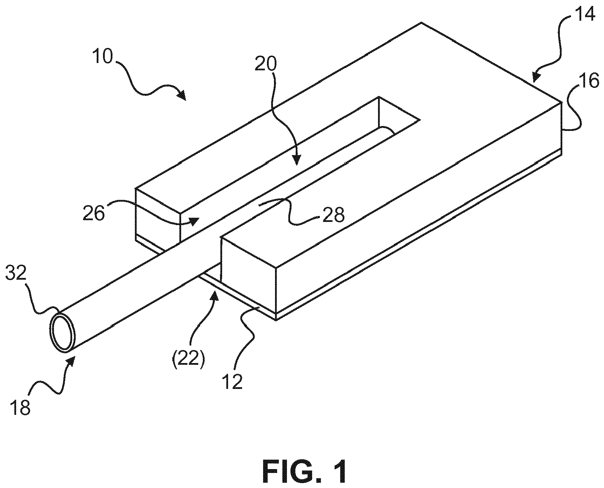

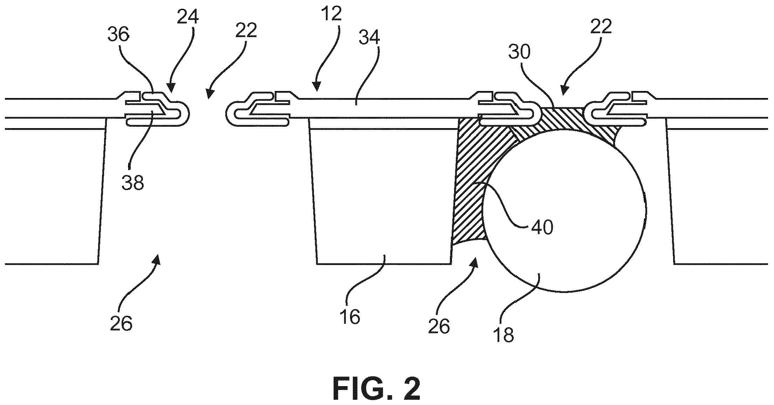

Fig. 1 shows an example of an electric component 10 of an interventional device. The component 10 comprises a flat carrier base 12, at least one electric circuit 14 provided on a substrate 16 and at least one electric wire 18 connected to the electric circuit 14 by a wire connection 20. The substrate 16 is attached to the carrier base 12. For the wire connection 20, the carrier base 12 is provided with at least one slit opening 22 (see also Fig. 2) that is provided at least partly with a conductive edge portion 24, which edge portion 24 is connected to the at least one electric circuit 14 (not shown in detail). The substrate 16 is provided with at least one recess 26 aligned with the location of the at least one slit opening 22 in the carrier base. An end portion 28 of the wire 18 is arranged in the at least one recess 26. The end portion of the wire is conductively coupled to the conductive edge portion by an electrically conductive material 30, e.g. soldering material and/or electrically conductive adhesive.

The recess 26 in the substrate is provided in a linear manner and can thus also be referred to as a slit recess. The recess 26 may be provided with an end wall section, e.g. formed by a U-formed enclosing substrate as shown in Fig. 1. It is noted that the substrate may have various configurations depending on the respective electrical circuit. The recess may as well be provided without end wall, as also shown in the following figures.

Fig. 2 shows a cross section of the electric component of Fig. 1. As indicated, the end 28 of the wire 18 is conductively coupled to the conductive edge portion 24 by a solder connection, e.g. solder between the conductive edge and at least the side of the wire facing the conductive edge. In addition, the solder may be provided at least partly surrounding and thus enclosing the end portion of the wire.

In Fig. 2, the cable 18 is inserted in the right recess 26, whereas the left recess 26 is shown without a cable for illustrative purposes.

The substrate of the electric circuit may be provided as one substrate portion or as several, segmented substrate part-portions, each mounted to the flat carrier base.

In an example, the wire is provided with a circumferentially enclosing electric insulation 32, which is removed at the end portion of the wire 18.

In an example, as shown in Fig. 2, the substrate of the at least one electric circuit 14 is attached to the carrier base 12 on a first side, e.g. as the lower side in Fig. 2, and the at least one electric wire 18 is connected to the carrier base also on the first side, e.g. also the lower side in Fig. 2. The wires are hence arranged on the same side as the substrate of the electric circuit. The wires are thus provided in-plane with the circuits, resulting in a flat component suitable for insertion into small lumens, e.g. small vessels.

In an example, provided as an option, the carrier 12 base comprises a flexible layer 34 and conductor paths attached to the flexible layer. For example, the carrier base is made from polyimide. The conductor paths are connected to the electric circuit. The conductor paths can also be referred to as contacts or circuit elements. In an example, the carrier base is multi-layered with at least two layers and the conductor paths are provided between the two layers.

In an example, provided as a further option but not further shown, the carrier base comprises a rigid layer.

In an example, the slit openings are provided as metalized slots in e.g. silicon contact pads. The conductive edge portions can also be referred to as bond pads with openings.

In Fig. 2, the conductive edge portions are provided as metal pads 36 clamped on edges 38 of the slit openings 22 of the carrier base 12. Further, the metal pads 36 are connected to the conductor paths (not shown in detail). In an example, the metal pads are provided as nickel/gold (Ni/Au) plating on aluminum (Al) pads.

In an example, provided as an option, the substrate 16 of the electric circuit 14 is a rigid substrate made from silicon.

The wire connection has two functions. First, the mechanical connection holds the connected wire in place. Second, the conductive connection provides the electric connection.

In an example, provided as an option, the electric wire 18 is mechanically coupled with an adhesive 40 to edge portions of the substrate forming the recess.

In the example with the removed insulation 32 and the coupling with the adhesive 40, the two functions, mechanical connection and conductive connection, are provided separated from each other, but in the same location.

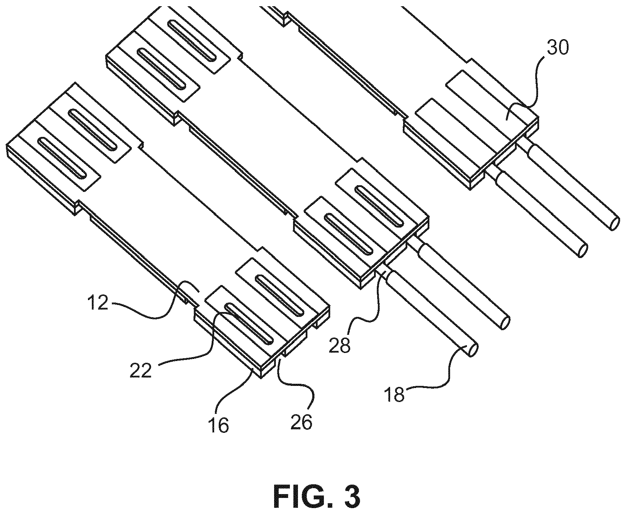

Fig. 3 shows manufacturing stages of an example of an electric component.

In the lower left, the carrier base 12 is shown with the slit opening 22 and the conductive edge portions 24. The substrate 16 with the recess 26 is below the carrier base 12.

As indicated more than one slit opening / recess arrangement can be provided. In Fig. 3, two parallel slits are shown, but also more than two, e.g. three, four, five or more can be provided.

In the lower middle, the cable 18 is inserted into the recess 26. As indicated, the insulation 32 is removed at the end portion 28 of the cable 18.

In the lower right, the soldering is applied via the slit openings 22 connecting the end portion 28 of the cable 18 with the conductive edge portions 24.

In Fig. 3, a further cable connection is indicated in the upper left part. This may be provided e.g. for connecting with further circuits, for example when forming an array of sensors.

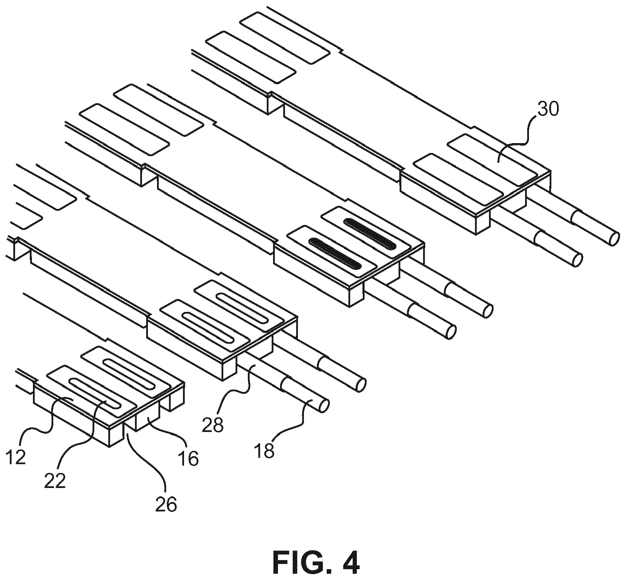

Fig. 4 shows manufacturing stages of another example of an electric component.

In the lower left, the carrier base 12 is shown with the slit opening 22 and the conductive edge portions 24. The substrate 16 with the recess 26 is below the carrier base 12. In the lower middle left, the cable 18 is inserted into the recess 26. For example, the cable is attached and hold in place with a glue.

In the lower middle right, provided as an option, the end portion 28 of the wire 18 is provided with the circumferentially enclosing electric insulation 32, which is removed in alignment with the respective slit opening 22 on a side of the wire facing the slit opening 22. The enclosing electric insulation 32 is thus only removed in the area of the slit opening 22. In an example, the electric insulation 32 is removed by laser, for example, once the end portion 28 of the wire is placed in the recess 26. In an example, the electric insulation 32 is removed after further attachment of the wire, e.g. by the glue. In another example, the electric insulation 32 is removed before further attachment.

In the lower right, the soldering is applied via the slit openings 22 connecting the end portion 28 of the cable 18 with the conductive edge portions 24.

In an example, the slit opening 22 is also provided by laser removing e.g. the polyimide layer, a glue (if present), and the wire insulation in the same laser removal process. In such workflow, the carrier base 12 may be provided without the slit openings 22 and the slit openings 22 are then provided during the third stage as shown in the lower middle right in Fig. 4.

In Fig. 4, further cable connections are indicated in the upper left part, as an option, e.g. when a plurality of further circuits is provided.

In an example, as shown in Fig. 3 and 4, the at least one recess 26 is arranged such that the at least one slit opening 22 reaches into the at least one recess 26.

Hence, overlapping recess and slit are provided. The slit opening 22 thus opens into the recess 26. The recess and the slit opening are aligned in an opening direction of the slit openings, i.e. so-to-speak aligned in a vertical direction. The first side can also be referred to as lower side or bottom side, and the recesses are arranged aligned below the slit openings provided in the carrier base that is arranged on an upper side of the substrate of the electric circuit.

In the example with aligned recess and slit, i.e. the overlapping recess and slit arrangement, the two functions, mechanical connection and conductive connection, are provided in an integrated manner e.g. by the solder connection.

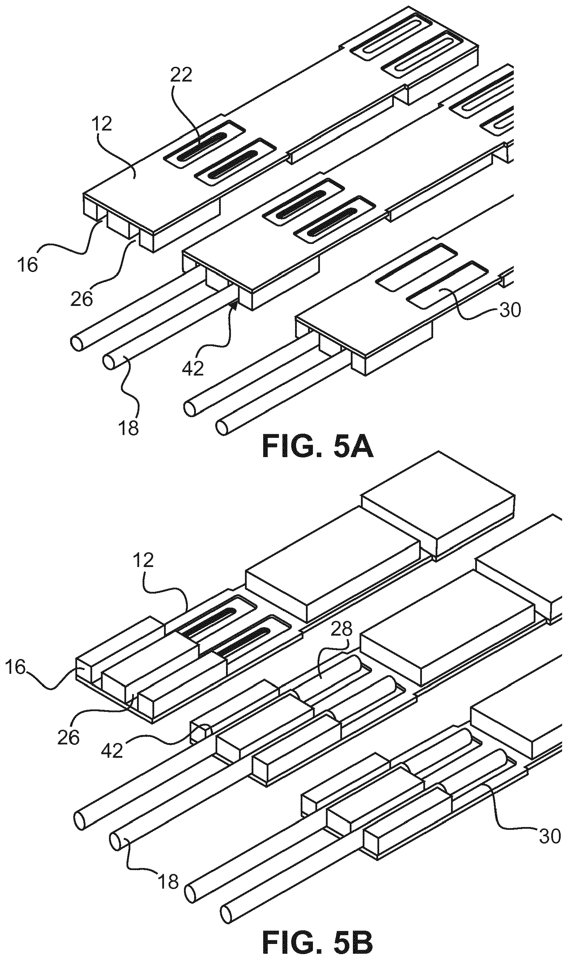

Fig. 5a shows manufacturing stages of a further example of an electric component in a first viewing direction. The at least one recess 26 is arranged in line with, but displaced to the at least one slit opening 22. As indicated in the lower middle in Fig. 5a, the wire first extends through the recess and then reaches the slit opening (see also lower middle in Fig. 5a). The wire is provided with a circumferential layer of the electric insulation 32 in a portion within the recess 26. Further, the wire is mechanically coupled with an adhesive 42 (not shown in detail) to edge portions of the substrate forming the recess 26. The recess is only provided in the first part of the wire overlapping with the substrate 16. The recess 26 is not provided below (Fig. 5a; or "above" in Fig. 5b) the slit opening 22.

In Fig. 5a, further cable connections are indicated in the upper right as an option.

Fig. 5b shows the manufacturing stages of Fig. 5a in a second, opposite viewing direction.

In Fig. 5b, the substrate 16 is indicated to be provided as several sub-segments, e.g. depending on the demands for the electrical circuit provided on, respectively also within the substrate.

In an example, the mechanical coupling is provided by an epoxy embedding.

The mechanical coupling provides a stress relief for the solder connection of the wire ends.

In an example, the adhesive is a glue, e.g. an epoxy glue.

In the example with displaced recess and slit, the two functions, mechanical connection and conductive connection, are provided separated from each other in two locations.

In an example, not further shown in detail, the at least one slit opening is arranged in line with the at least one recess, wherein the recess is having a longer extension than the slit opening such that a first part of the recess is provided with the slit opening in the carrier base and a second part is provided without a slit opening in the carrier base. Further, the wire is provided with an insulated section having a circumferential layer of electric insulation in a portion within the second part of the recess, in which insulated section the wire is mechanically coupled with an adhesive to edge portions of the substrate forming the recess. Still further, the wire is provided with a stripped end section without insulation in a portion within the first part of the recess, in which stripped end section the wire is connected by the soldering. The recess is thus longer than the slits such that the wire is separately mechanically connected and conductively connected.

The electric component is provided as at least one of the group of a sensor, a measurement unit and an application unit for an interventional device. For example, the electric component is a capacitive micromachined ultrasonic transducer (CMUT) or CMUT transducer array. The CMUT is for example used for at least one of an ultrasound imaging on an intravascular ultrasound (IVUS) device, a flow sensing and pressure sensing on a physiological measurement device (e.g. flow-wire, pressure-wire).

In an option, the electric component comprises a plurality of electric circuits. A plurality of interconnecting wires is provided between the electric circuits. Further, the interconnecting wires are connected by wire connections with the slit openings in the carrier base and the recesses in the substrates.

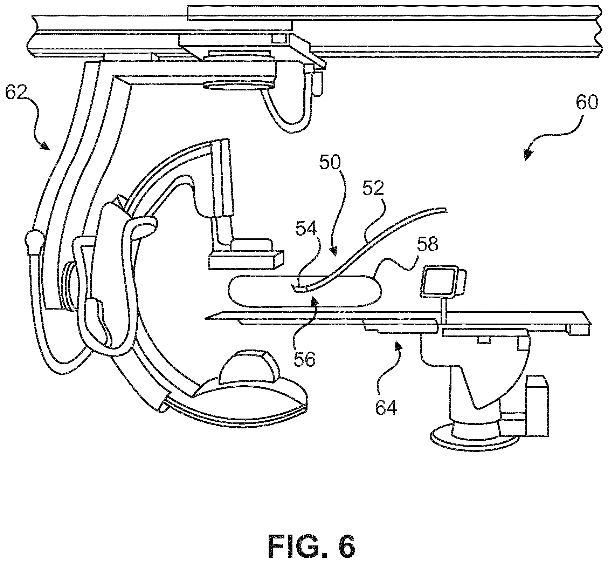

Fig. 6 shows an example of an interventional device 50 for at least partly insertion into a subject. The device 50 comprises an elongated main body structure 52 and an electric component 54 provided according to one of the preceding examples. The main body structure comprises a distal part 56 for insertion into a subject 58. Further, the electric component is attached to the distal part of the main body structure.

In an example, the interventional device is provided as a catheter or guidewire. In another example, the interventional device is provided as an ultrasound probe for intravascular ultrasound imaging.

The term "subject" may also be referred to as individual. The "subject" may further also be referred to as patient, although it is noted that this term does not indicate whether any illness or disease is actually present with the subject.

Fig. 6 shows the interventional device 50 in the context of a system 60 for medical intervention, as an example. The system 60 for medical intervention may comprise a medical imaging arrangement 62, which for example is shown as a C-arm based X-ray imaging device. The subject 58 may be arranged on a patient support 64.

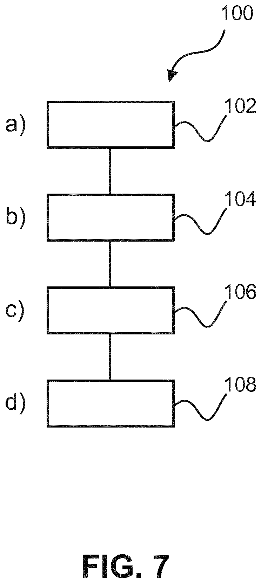

Fig. 7 shows steps of an example of a method 100 for manufacturing an electric component of an interventional device

In a first step 102, also referred to as step a), a flat carrier base with at least one electric circuit provided on a substrate is provided. The substrate circuit is attached to the carrier base. Further, the carrier base is provided with at least one slit opening that is provided at least partly with a conductive edge portion, which edge portion is connected to the at least one electric circuit. Still further, the substrate is provided with at least one recess aligned with the location of the at least one slit opening in the carrier base.

In a second step 104, also referred to as step b), at least one electric wire for connection with the electric circuit is provided.

In a third step 106, also referred to as step c), an end portion of the at least one electric wire is arranged in the at least one recess.

In a fourth step 108, also referred to as step d), the end portion of the wire is attached to the conductive edge portion to conductively couple the end portion of the wire and the conductive edge portion. This can be for example be done by soldering or gluing with electrically conductive material (e.g. silver glue).

In an example, the substrate with the electric circuit is attached to the carrier base on a first side and the at least one electric wire is connected to the carrier base also on the first side. The soldering takes place through the at least one slit opening from a second side of the carrier base, the second side being opposite to the first side.

In an option, not shown in detail, in step c), the end portion of the wire is provided with a circumferentially enclosing electric insulation. Further, before step d), the insulation is removed in alignment with the respective slit opening on a side of the wire facing the slit opening.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An electric component (10) of an interventional device, the component comprising:
- a flat carrier base (12);
- at least one electric circuit (14) provided on a substrate (16); and
- at least one electric wire (18) connected to the electric circuit by a wire connection (20);
wherein the substrate is attached to the carrier base;
wherein, for the wire connection, the carrier base is provided with at least one opening (22) that is provided at least partly with a conductive edge portion (24), which edge portion is connected to the at least one electric circuit;
wherein the substrate is provided with at least one recess (26) aligned with the location of the at least one opening in the carrier base; and
wherein an end portion (28) of the wire is arranged in the at least one recess; and wherein the end portion of the wire is conductively coupled to the conductive edge portion by an electrically conductive material (30).

2. Electric component according to claim 1, wherein the substrate of the at least one electric circuit is attached to the carrier base on a first side and the at least one electric wire is connected to the carrier base also on the first side.

3. Electric component according to claim 1 or 2, wherein the carrier base comprises a flexible layer (34) and conductor paths attached to the flexible layer.

4. Electric component according to claim 1, 2 or 3, wherein the conductive edge portions are provided as metal pads (36) clamped on edges (38) of the openings of the carrier base; and
wherein the metal pads are connected to the conductor paths.

5. Electric component according to one of the preceding claims, wherein the substrate of the electric circuit is a rigid substrate made from silicon.

6. Electric component according to one of the preceding claims, wherein the end portion of the wire is provided with a circumferentially enclosing electric insulation (32) which is removed in alignment with the respective opening on a side of the wire facing the opening.

7. Electric component according to one of the preceding claims, wherein the wire is mechanically coupled with an adhesive (40) to edge portions of the substrate forming the recess.

8. Electric component according to one of the claims 1 to 7, wherein the at least one recess is arranged such that the at least one opening reaches into the at least one recess.

9. Electric component according to one of the claims 1 to 7, wherein the at least one recess is arranged in line with, but displaced to the at least one opening; wherein the wire first extends through the recess and then reaches the opening; and
wherein the wire is provided with a circumferential layer of electric insulation in a portion within the recess; and wherein the wire is mechanically coupled with an adhesive to edge portions of the substrate forming the recess.

10. Electric component according to one of the claims 1 to 7, wherein the at least one opening is arranged in line with the at least one recess, wherein the recess is having a longer extension than the opening such that a first part of the recess is provided with the opening in the carrier base and a second part is provided without an opening in the carrier base;
wherein the wire is provided with an insulated section having a circumferential layer of electric insulation in a portion within the second part of the recess, in which the insulated section of the wire is mechanically coupled with an adhesive to edge portions of the substrate forming the recess; and
wherein the wire is provided with a stripped end portion without insulation in a portion within the first part of the recess, in which the stripped end portion of the wire is connected by the soldering material.

11. Electric component according to one of the preceding claims, wherein the electric component is provided as at least one of the group of a sensor, a measurement unit and an application unit for an interventional device.

12. Electric component according to one of the preceding claims, wherein the electric component comprises a plurality of electric circuits;
wherein a plurality of interconnecting wires is provided between the electric circuits; and
wherein the interconnecting wires are connected by wire connections with the openings in the carrier base and the recesses in the substrates.

13. An interventional device (50) for at least partly insertion into a body of a subject, the device comprising:
- an elongated main body structure (52); and
- an electric component (54) provided according to one of the preceding claims;
wherein the main body structure comprises a distal part for insertion into the body of the subject; and
wherein the electric component is attached to the distal part of the elongated main body structure.

14. A method (100) of manufacturing an electric component of an interventional device, the method comprising the following steps:
a) providing (102) a flat carrier base with at least one electric circuit provided on a substrate, the substrate circuit being attached to the carrier base;
wherein the carrier base is provided with at least one opening that is provided at least partly with a conductive edge portion, which edge portion is connected to the at least one electric circuit; and
wherein the substrate is provided with at least one recess aligned with the location of the at least one opening in the carrier base;
b) providing (104) at least one electric wire for connection with the electric circuit;
c) arranging (106) an end portion of the at least one electric wire in the at least one recess; and
d) attaching (108) the end portion of the wire to the conductive edge portion to conductively couple the end portion of the wire and the conductive edge portion.

15. Method according to claim 14, wherein, in step c), the end portion of the wire is provided with a circumferentially enclosing electric insulation; and
wherein, before step d), the insulation is removed in alignment with the respective opening on a side of the wire facing the opening.
